# FASCICULE DE BREVET EUROPEEN

(11) **EP 1 000 952 B1**
(45) Date de publication et mention de la délivrance du brevet: **09.03.2011**
(21) Numéro de dépôt: 99402523.7
(22) Date de dépôt: 14.10.1999
(51) Int. Cl.: C07H 17/08, A61K 31/70, A61P 31/04

(54) **Nouveaux dérivés 2-halogénés de 5-0-désosaminylérythronolide A, leur procédé de préparation et leur application comme médicaments**
Neue 2-Halogen-Derivate von 5-O-Desosaminylerythronolid A, Verfahren zu deren Herstellung sowie deren Verwendung als Arzneimittel
New 2-halogenated derivatives of 5-O-deosaminylerythronolide A, process for making them as well as their use as medicines

(30) Priorité: 15.10.1998 FR 9812937
(43) Date de publication de la demande: 17.05.2000
(73) Titulaire: Aventis Pharma S.A., 92160 Antony (FR)
(72) Inventeur: Agouridas, Constantin, 94130 Nogent sur Marne (FR); Denis, Alexis, 75011 Paris (FR); Bretin, Francois, 77330 Ozoir la Ferriere (FR); Fromentin, Claude, 75019 Paris (FR)
(74) Mandataire: Le Coupanec, Pascale A.M.P.

(56) Documents cités:
- EP-A- 0 487 411
- WO-A-99/21871
- FR-A- 2 742 757

## Description

La présente invention concerne de nouveaux dérivés 2-halogène de 5-O-désosaminylérythronolide A, leur procédé de préparation et leur application comme médicaments. L'invention a pour objet les composés de formule (I) : 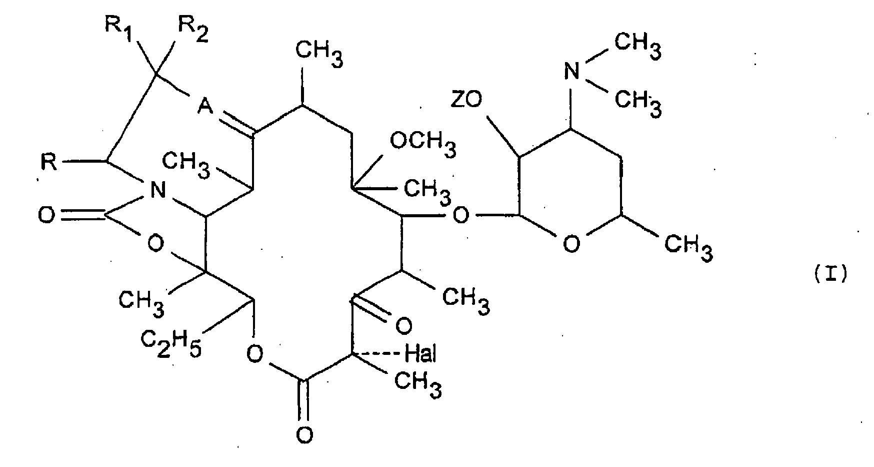 dans lesquels A représente un atome d'azote ou un groupement N->O, R₁ et R₂, identiques ou différents représentent un atome d'hydrogène ou un radical alkyle, renfermant jusqu'à 18 atomes de carbone, R représente un atome d'hydrogène, un radical (CH₂)ₘOB dans lequel m représente un nombre entier compris entre 1 et 8 et B représente un atome d'hydrogène ou un radical COAr ou un radical (CH₂)ₙAr, n représentant un nombre entier compris entre 1 et 8 et Ar représentant un radical aryle ou hétéroaryle mono ou polycyclique et Z représente un atome d'hydrogène ou le reste d'un radical acyle renfermant jusqu'à 18 atomes de carbone, ainsi que leurs sels d'addition avec les acides.

On connaît déjà dans l'art antérieur et notamment dans les demandes EP 0 487 411 A et FR 2 742 757A des dérivés de l'érythromycine. Cependant, ces dérivés de l'art antérieur ne comprennent pas un groupement reliant l'atome d'azote du carbamate et l'atome d'azote représenté par A dans la présente demande, et formant ainsi un cycle.

Parmi les sels d'addition avec les acides, on peut citer les sels formés avec les acides acétique, propionique, trifluoroacétique, maléïque, tartrique, méthanesulfonique, benzènesulfonique, p-toluènesulfonique et spécialement les acides stéarique, éthylsuccinique ou laurylsulfonique.

Le radical alkyle est de préférence un radical méthyle, éthyle, propyle, isopropyle, n-butyle, isobutyle, terbutyle, décyle ou dodécyle.

Le radical aryle peut être un radical phényle ou naphtyle. Le radical hétéro aryle peut être un radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle, benzofurannyle, benzothiazyle ou quinoléinyle.

Lorsque les radicaux mentionnés sont substitués ils peuvent l'être par un ou plusieurs des radicaux suivants : les radicaux hydroxyle, les atomes d'halogène, les radicaux NO₂, les radicaux C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle renfermant jusqu'à 12 atomes du carbone éventuellement substitués par un ou plusieurs atomes d'halogène, le radical 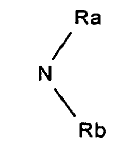 Ra et Rb identiques ou différents représentent un atome d'hydrogène ou un radical alkyle renfermant jusqu'à 12 atomes de carbone, le radical 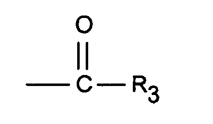 R₃ représentant un radical alkyle, renfermant jusqu'à 12 atomes de carbone, ou un radical aryle ou hétéroaryle éventuellement substitué, les radicaux aryle, O-aryle ou S-aryle carboxyliques, ou aryle, O-aryle ou S-aryle hétérocycliques à 5 ou 6 chaînons comportant un ou plusieurs hétéroatomes, éventuellement substitués par un ou plusieurs des substituants mentionnés ci-dessus.

Hal représente de préférence le fluor ou le chlore. Lorsque l'un des radicaux est substitué par un atome d'halogène, il s'agit de préférence du fluor, du chlore ou du brome.

L'invention a plus particulièrement pour objet les composés de formule (I) dans lesquels R₁ et R₂ représentent un atome d'hydrogène, ceux dans lesquels A représente un atome d'azote, ceux dans lesquels Hal représente un atome de fluor, ceux dans lesquels R représente un atome d'hydrogène, ainsi que ceux dans lesquels R représente un radical CH₂OH.

L'invention a plus particulièrement pour objet les composés dont la préparation est donnée ci-après dans la partie expérimentale.

Les produits de formule générale (I) possèdent une très bonne activité antibiotique sur les bactéries gram^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples dont la préparation est donnée ci-après et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

L'invention a également pour objet un procédé caractérisé en ce que l'on soumet un composé de formule (II) : 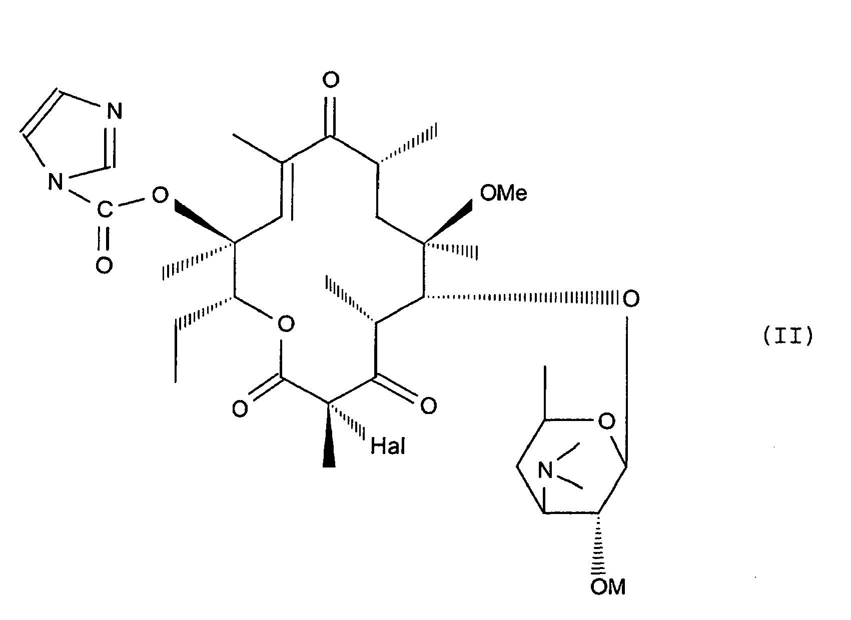 dans laquelle Hal représente un atome d'halogène, OM représente un groupement hydroxyle bloqué, à l'action d'un composé de formule (III) : 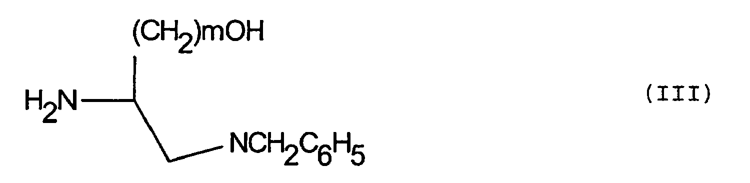 dans laquelle m représente un nombre entier compris entre 1 et 8, pour obtenir le composé de formule (IV) : 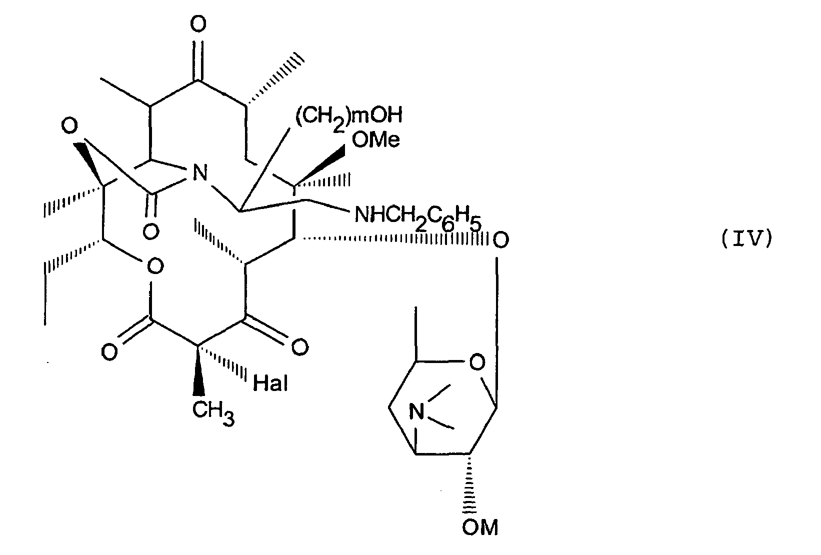 puis libère l'hydroxyle en 2' pour obtenir le composé de formule (V) : 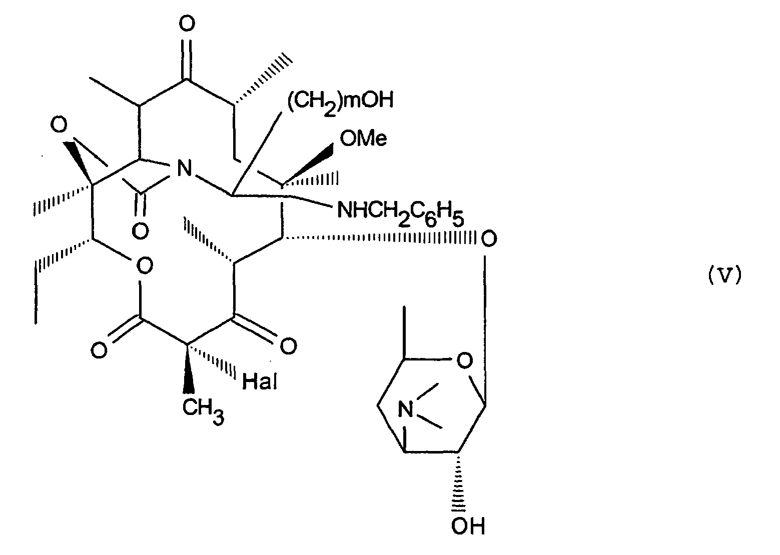 que l'on soumet à l'action d'un agent de débenzylation, pour obtenir le composé de formule (VI) : 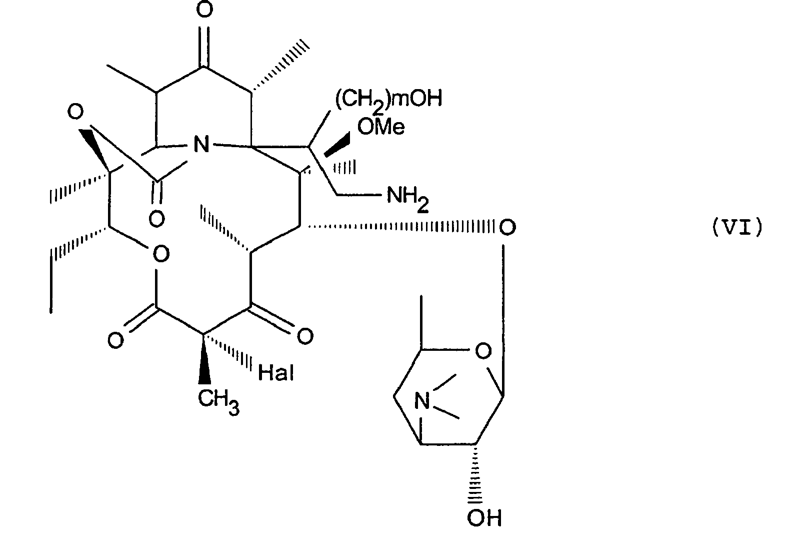 que l'on soumet à l'action d'un agent de cyclisation pour obtenir le composé de formule (IA) : 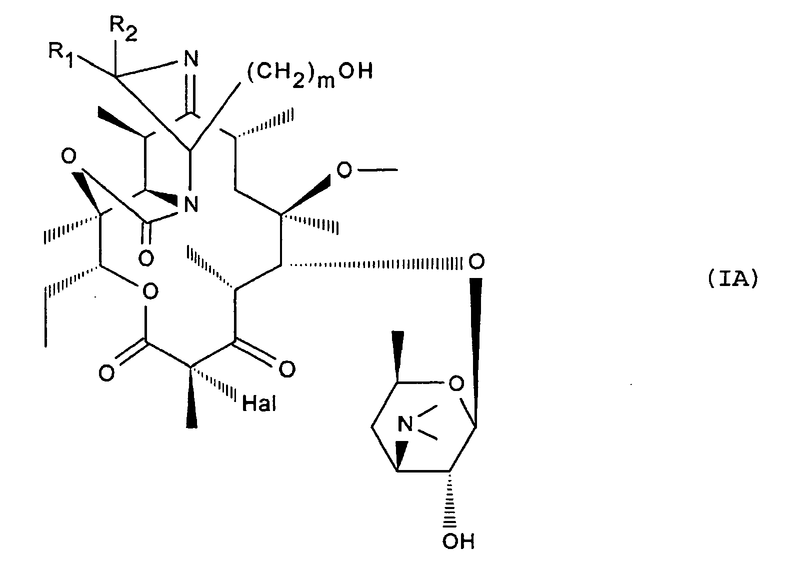 dans lequel R représente un radical (CH₂)ₘOH, que l'on soumet à l'action d'un agent d'alkylation ou d'acylation du groupement (CH₂)ₘOH pour obtenir le composé de formule (IB) correspondant das lequel B représente un groupement COAr ou (CH₂)ₙAr, puis, si désiré estérifie le groupement OH en 2' correspondant dans lequel B représente un groupement COAr ou (CH₂₎ₙAr, puis, si désiré estérifie le groupement OH en 2' et/ou soumet à l'action d'un acide pour obtenir un sel du composé de formule (I) obtenu.

Les produits de formule (II) utilisés comme produits de départ sont décrits et revendiqués dans la demande de brevet français 9804366 déposée le 8 avril 1998. Il est décrit ci-après un exemple détaillé de préparation de composés de formule (I) dans le cas où Hal représente un atome de fluor. Ce procédé peut être schématisé comme suit : on soumet le composé A : 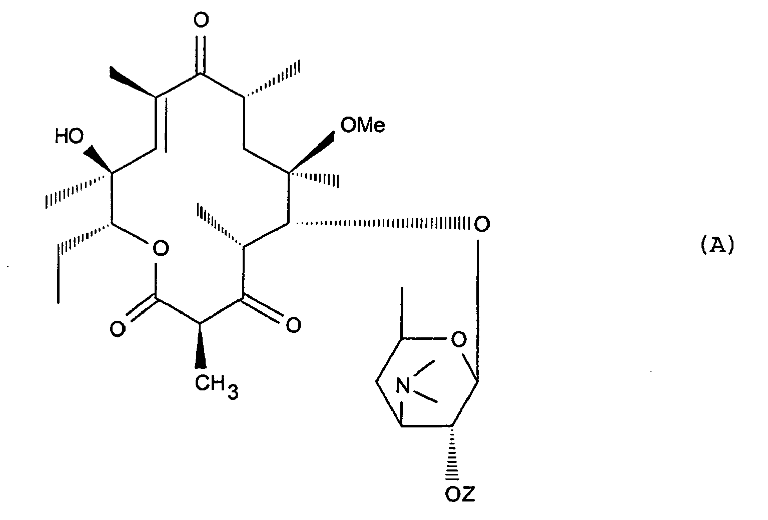 dans laquelle OZ représente un radical OH libre ou protégé, à l'action d'un agent de fluoration pour obtenir le composé de formule (B) correspondant : 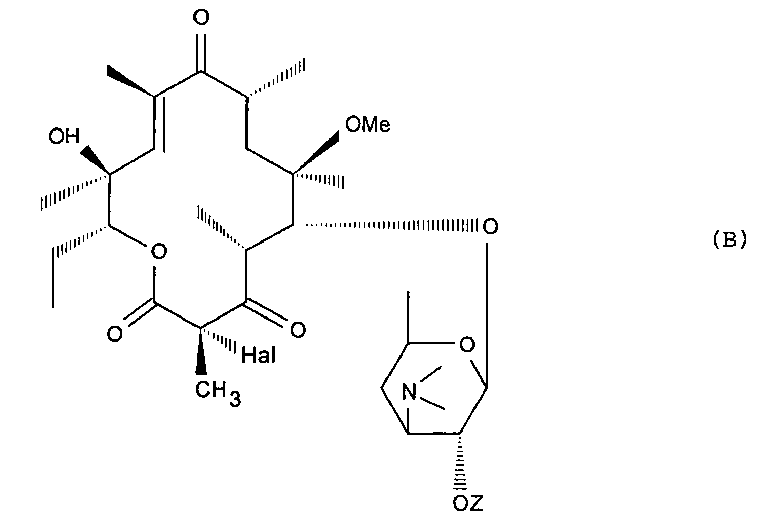 que l'on soumet à l'action du carbonyldiimidazole, pour obtenir le composé de formule (II) correspondant.

Les autres composés de formule (II) peuvent être préparés par analogie avec le procédé décrit ci-après de façon détaillée.

Dans un mode de réalisation préféré du procédé de l'invention :
- le radical OZ est un radical acétyle ou benzyle,
- la libération de l'hydroxyle à partir de OZ est réalisée par méthanolyse,
- la débenzylation est réalisée par hydrogénation (par exemple avec du palladium sur charbon en présence de formiate d'ammonium au reflux du méthanol),
- la cyclisation est réalisée au reflux de l'éthanol en présence d'acide acétique,
- l'alkylation ou l'acylation du radical (CH₂)ₙOH est réalisée selon les méthodes classiques.

Les produits intermédiaires (IV), (V) et (VI) mis en oeuvre au cours du procédé de l'invention sont des produits nouveaux et sont en eux-mêmes un objet de la présente invention.

Les produits de formule (I) peuvent également être préparés par halogénation directe des produits non halogénés correspondants ; l'invention a pour objet un procédé caractérisé en ce que l'on soumet un composé de formule (IIIA) : 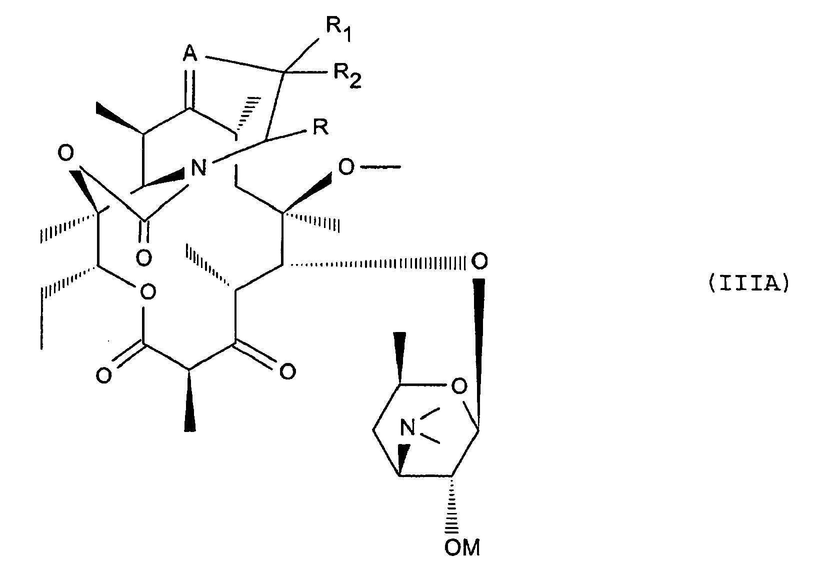 dans laquelle A, R, R₁ et R₂ conservent leur signification précédente et OM représente un groupement hydroxyle bloqué, à l'action d'un agent d'halogénation pour obtenir le composé de formule (IB) : 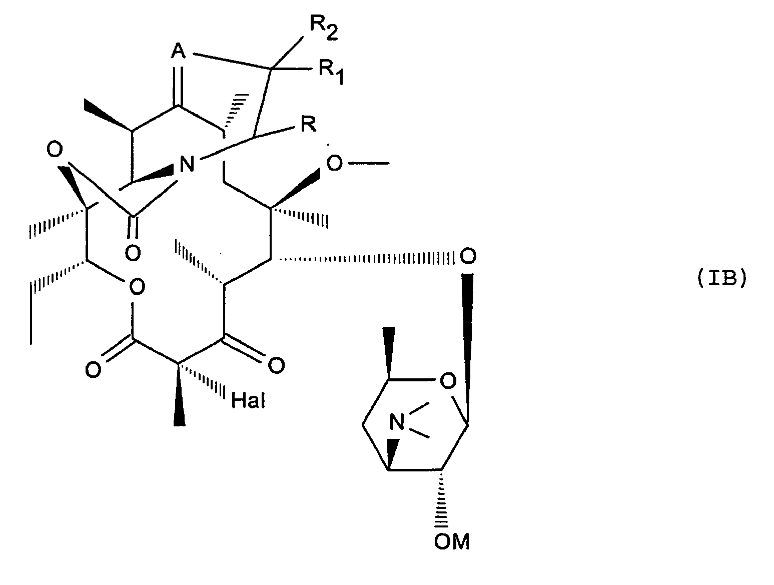 que l'on soumet si désiré, à l'action d'un agent de libération du groupement hydroxyle en 2', pour obtenir le composé de formule (I) correspondant dans lequel Z est un atome d'hydrogène que l'on soumet, si désiré à l'action d'un agent d'estérification du groupe OH en 2' ou à l'action d'un acide pour en former le sel.

Dans un mode de réalisation préféré, l'agent d'halogénation est le bisphénylsulfonylimide de formule :

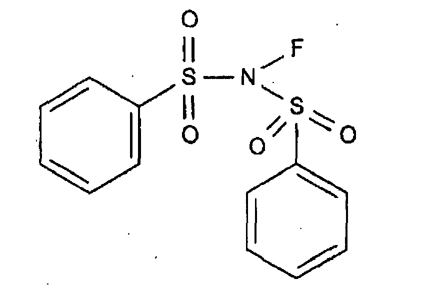

### EXEMPLE 1 : [3aS-(3aR*,4S*,7R*,9S*,10S*,11S*,13S*,15S*,-15aS*)] -4-éthyl-7-fluoro-3a,4,10,11,12,13,15,15a-octahydro-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(9H)-trione (référence)

### Stade A : [3aS-(3aR*,4S*,7S*,9S*,10S*,11S*,13S*,15S*,15aS*)] -4-éthyl-3a,4,10,11,12,13,15,15a-octahydro-11-méthoxy-3a,7,9,11,13,15-hexaméthyl-10-[[3,4,6-tridéoxy-3-(diméthylamino)-2-O-(triméthylsilyl)-.béta.-D-xylo-hexopyranosyl]oxy]-14,1-(nitriloéthano)-2H-oxacyclotétradécino[4,3-d]oxazole-2,6,8(7H,9H)-trione

On agite pendant 5 minutes, un mélange de 0,9835 g de [3aS-(3aR*,4S*,7S*,9S*,10S*,11S*,13S*,15S*,15aS*)] -4-éthyl-3a,4,10,11,12,13,15,15a-octahydro-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloêthano)-2H-oxacyelo-Cétradécino[4,3-d]oxazole-2,6,8(7H,9H)-trione (EP 0638585) et 9,8 cm³ de THF. On ajoute 105 mg d'imidazole et 0,327 cm³ de hexaméthylsilylamine[(CH₃)₃Si]₂NH. On agite pendant 5 jours durant lesquel on additionne 2 x 0,2 éq de 3-pyrazolamine et 2 x 0,2 éq d'hexaméthylsilylamine. On amène à sec, reprend au chlorure de méthylène et ajoute 30 cm³ d'une solution de dihydrogenophosphate de sodium. On agite pendant 15 minutes, décante, extrait la phase aqueuse au chlorure de méthylène. On rassemble les phases chlorométhyléniques, sèche, filtre et évapore. On obtient 1,2259 g de produit recherché.

### Stade B _{:} [3aS-(3aR*,4S*,7S*,9S*,10S*,11S*,13S*,15S*,15aS*)] -4-éthyl-7-fluoro-3a,4,10,11,12,13,15,15a-octahydro-11-méthoxy-3a,7,9,11,13,15-hexaméthyl-10-[[3,4,6-tridéoxy-3-(diméthylamino)-2-0-(triméthylsilyl)-.béta.-D-xylohexopyranosyl]oxy]-14,1-(nitriloéthano)-2H-oxacyclotétradécino[4,3-d]oxazole-2,6,8(7H,9H)-trione

On refroidit à -10°C une solution renfermant 1,1003 g de produit du stade A et 11 cm³ de THF. On ajoute 1,86 cm³ de terbutylate de potassium dans le THF. On agite pendant 5 minutes et ajoute 0,588 g de

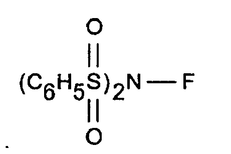

On agite 10 minutes à -10°C et laisse revenir à la température ambiante. On agite à la température ambiante pendant 1 h 30. On filtre, rince le précipité obtenu à l'acétate d'éthyle : Le filtrat est concentré et repris avec 10 cm³ d'acétate d'éthyle, 10 ml d'eau et 5 ml d'une solution aqueuse d'ammoniaque à 20 %. On agite 10 minutes, décante, lave à l'eau. On extrait à l'acétate d'éthyle, rassemble les phases organiques, les sèche, filtre et évapore à sec. On obtient 1,1067 g de produit recherché.

### Stade C : [3aS-(3aR*,4S*,7R*,9S*,10S*,11S*,13S*,15S*,15aS*)] -4-éthyl-7-fluoro-3a,4,10,11,12,13,15,15a-octahydro-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(9H)-trione

On ajoute 1,13 cm³ d'une solution de fluorure de tétrabutylammonium dans le THF dans une solution renfermant 0,55 g de produit du stade B et 5,5 cm³ de THF. On agite pendant 4 h 30, le solvant est évaporé et le résidu repris dans 5 cm³ d'acétate d'éthyle, 5 ml d'eau et 2 cm³ d'ne solution d'ammoniaque à 20 %. On agite pendant 15 minutes, décante. On extrait la phase aqueuse à l'acétate d'éthyle. On lave à l'eau, réextrait la phase aqueuse. On rassemble les phases organiques, sèche, filtre et évapore à sec. On obtient 0,4134 g de produit recherché.

### EXEMPLE 2 : (3aS,4R,7S,9R,10R,11R,13R,15R,15aR,18S)-4-etyl-7-fluoro-3a,4,10,11,12,13,15,15a-octahydro-18-(hydroxyméthyl)-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione

### Stade A : 11,12-didéoxy-3-de[(2,6-didéoxy-3-C-méthyl-3-O-méthyl-.alpha.-L-ribo-hexopyranosyl)oxy]-2-fluoro-6-O-méthyl-3-oxo-12,11-[oxycarbonyl[[(2R)-1-hydroxy-3-[(phénylméthyl) amino]-2-propyl]imino]-2'-acétoxy

On introduit 6,7 g du produit de la préparation I dans une solution renfermant 8,33 g de (R)-2-amino-3-[(phénylméthyl)amino]-1-propanol, 67 cm³ d'acétonitrile et 6,7 cm³ d'eau. On porte le mélange réactionnel à 55°C, et le maintient à cette température pendant 21 heures. On verse le mélange réactionnel sur un mélange eau-acétate d'éthyle. On décante. On extrait à l'acétate d'éthyle. On sèche, filtre et évapore. On obtient 10,7 g de produit.

### Stade B : 11,12-didéoxy-3-de[(2,6-didéoxy-3-C-méthyl-3-O-méthyl-.alpha.-L-ribo-hexopyranosyl)oxy]-2-fluoro-6-O-méthyl-3-oxo-12,11-[oxycarbonyl[[(2R)-1-hydroxy-3-[(phénylméthyl) amino]-2-propyl]imino]-érythromycine

On ajoute 107 cm³ de méthanol aux 10,7 g du produit du stade précédent. On agite pendant 15 heures à température ambiante, évapore le méthanol et amène à sec. On obtient 9,47 g de produit recherché brut que l'on purifie par 2 chromatographies successives en éluant avec le mélange chlorure de méthylène/méthanol/ammoniaque (96-4-0,4) puis en éluant avec le mélange acétate d'éthyle/ triéthylamine. On obtient 2,66 g de produit recherché.

### Stade C : 11,12-didéoxy-3-de[(2,6-didéoxy-3-C-méthyl-3-O-méthyl-.alpha.-L-ribo-hexopyranosyl)oxy]-2-fluoro-6-O-méthyl-3-oxo-12,11-[oxycarbonyl-[((2R)-1-amino-3-hydroxy-2-propyl) imino]]-érythromycine

On mélange 0,8 g du produit du stade précédent, 8 cm³ de méthanol, 315 mg de formiate d'ammonium et 800 mg de palladium sur charbon. Le mélange réactionnel est porté au reflux pendant 4 heures et demi sous hydrogène. On laisse revenir à la température ambiante puis filtre. Le filtrat est concentré sous pression réduite et l'on obtient 660 mg de produit que l'on reprend par 20 cm³ d'acétate d'éthyle. On verse sur une solution d'ammoniaque à 20 %. On agite, décante et extrait à l'acétate d'éthyle. On sèche et filtre. On obtient 660 mg de produit.

### Stade D : (3aS,4R,7S,9R,10R,11R,13R,15R,15aR,18S)-4-etyl-7-fluoro-3a,4,10,11,12,13,15,15a-octahydro-18-(hydroxyméthyl)-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione

On porte au reflux 0,3795 g du produit du stade précédent, 4 cm³ d'éthanol et 62 µl d'acide acétique. On maintient le mélange réactionnel sous agitation au reflux pendant 6 jours. On laisse revenir à la température ambiante. On concentre sous pression réduite, reprend à l'acétate d'éthyle et verse sur une solution d'ammoniaque à 20%. On agite pendant 15 minutes, décante, extrait à l'acétate d'éthyle, sèche, filtre, rince et évapore. On obtient 0,304 g de produit que l'on purifie par chromatographie sur silice en éluant avec le mélange chloroforme/isopropanol/ammoniaque (90-10-0,4). On obtient 88 mg de produit.

### Préparation 1 : 2'-acétoxy 2α-fluoro de 12-(oxycarbonyl-imidazol)-11-déoxy-10,11-didéhydro-3-de[2,6-didéoxy-3-C-méthyl-3-O-méthyl-α-L-ribohexopyraaosyl)oxy]6-O-mëthyl-3-oxo-érythromycine

### Stade A : 11-déoxy 10,11-didéhydro 3-de[(2,6-didéoxy 3-O-méthyl α-L-ribohexopyranosyl) oxy] 6-O-méthyl 3-oxo érythromycine.

On agite pendant 44 heures un mélange de 8,722 g de 2'-acétate de 11-déoxy 10,11-didéhydro 3-de[(2,6-didéoxy 3-O-méthyl α-L-ribohexopyranosyl) oxy] 6-O-méthyl 3-oxo érythromycine (EP 596802) et 350 ml de méthanol anhydre. On évapore, reprend au chlorure de méthylène, sèche et obtient 8,794 g du produit recherché.

### Stade B : 2'-triméthylsilyloxy de 11-déoxy 10,11-didéhydro 3-de[(2,6-didéoxy 3-O-méthyl α-L-ribohexopyranosyl) oxy] 6-O-méthyl 3-oxo érythromycine.

On agite à température ambiante pendant 4 jours un mélange renfermant 3,08 g du produit du stade précédent, 340 mg d'imidazole, 32 ml de THF anhydre et 1,06 ml d'hexaméthyl-disilylazane. On évapore à sec, reprend avec un mélange de 60 ml de chlorure de méthylène et de 60 ml d'une solution aqueuse de phosphate acide de sodium 0,5 M. On maintient le mélange sous agitation pendant 15 minutes, décante, extrait au chlorure de méthylène, sèche et évapore à sec. On obtient 3,345 g du produit recherché.

### Stade C : 2'-triméthylsilyloxy 2a-fluoro de 11-déoxy 10,11-didéhydro 3-de[(2,6-didéoxy 3-O-méthyl α-L-ribohexopyranosyl) oxy] 6-O-méthyl 3-oxo érythromycine.

On ajoute à -12°C sous atmosphère d'argon 1,24 ml d'une solution de terbutylate de potassium dans le THF 0,97M dans une solution renfermant 668 mg de 2'-triméthylsilyloxy de 11-déoxy 10,11-didéhydro 3-de[(2,6-didéoxy 3-O-méthyl α-L-ribohexopyrasonyl) oxy] 6-O-méthyl 3-oxo érythromycine et 6,7 ml de THF anhydre. On agite 5 minutes et ajoute 378 mg de N-fluoro dibenzènesulfonimide. On agite 10 minutes à -12°C et laisse revenir à la température ambiante pendant 1 heure 30 minutes. On effectue les opérations d'isolation et purification et obtient 695 mg du produit recherché.

### Stade D : 2α-fluoro de 11-déoxy 10,11-didéhydro 3-de[(2,6-didéoxy 3-C-méthyl 3-O-méthyl α-L-ribohexopyranosyl) oxy] 6-O-méthyl 3-oxo érythromycine.

On agite pendant 3 heures 30 minutes un mélange de 5,476 g de produit du stade précédent, 50 ml de THF et 11,2 ml de fluorure de tétrabutylammonium 1M dans le THF. On évapore le solvant et ajoute 37 ml d'acétate d'éthyle, 37 ml d'eau et 7,5 ml d'ammoniaque à 20%. On agite 10 minutes, décante, extrait à l'acétate d'éthyle, sèche, filtre et concentre à sec le filtrat. On chromatographie le produit obtenu sur silice en éluant avec le mélange CH₂Cl₂-MeOH ammoniaqué 99-1, puis 98-2, 97-3, 96-4, 95-5. On obtient 2,452 g de produit recherché.

### Stade E : 2'-acétoxy 2α-fluoro de 11-déoxy 10,11-didéhydro 3-de[(2,6-didéoxy 3-C-méthyl 3-O-méthyl α-L-ribohexopyrasonyl) oxy] 6-O-méthyl 3-oxo érythromycine.

On maintient sous agitation pendant 3 heures 1,02 g de produit du stade A, 10 ml de chlorure de méthylène et 241 µl d'anhydride acétique. On évapore et ajoute 10 ml d'eau et 10 ml d'acétate d'éthyle. On laisse 1 heure à la température ambiante sous agitation, décante, sèche et évapore. On obtient 1,01 g de produit recherché.

### Stade F : 2'-acétoxy 2α-fluoro de 12-(oxycarbonylimidazol) 11-déoxy 10,11-didéhydro 3-de[(2,6-didéoxy 3-C-méthyl 3-O-méthyl α-L-ribohexopyranosyl) oxy] 6-O-méthyl 3-oxo érythromycine.

On ajoute à 0°C 0,388 g de carbonyldiimidazole et 24 µl de DBU dans une solution renfermant 1,01 g du produit du stade précédent et 10 ml de THF anhydre.On maintient le mélange réactionnel sous agitation à 0°C pendant 19 heures. On évapore le THF et ajoute 10 ml d'eau et 10 ml d'acétate d'éthyle. On maintient le mélange réactionnel sous agitation pendant 10 minutes, extrait, sèche et évapore. On obtient 0,902 g de produit recherché brut que l'on chromatographie en éluant avec un mélange acétate d'éthyle-triéthylamine 96-4. On obtient 0,573 g de produit recherché.

### Exemple 3 : (3aS,4R,7S,9R,10R,11R,13R,15R,15aR,18S)-4-etyl-7-fluoro-3a,4,10,11,12,13,15,15a-octahydro-18-[[[(4-quinoleinyl)carbonyl]oxy]méthyl]-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione.

### Stade A : (3aS,4R,7S,9R,10R,11R,13R,15R,15aR,18S)-4-etyl-7-fluoro-3a,4,10,11,12,13,15,15a-octahydro-18-(hydroxyméthyl)-11-méthoxy-3a,7,9,11,13,15-hexaméthyl-10-[[2-O-acétyl-3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione.

On agite à la température ambiante pendant 20 heures 299 mg du produit de l'exemple 2, 3cm³ d'acétate d'éthyle et 46 µl d'anhydride acétique. On verse sur une solution saturée d'ammoniaque à 20 %, on agite 20 minutes, décante et extrait à l'acétate d'éthyle. On sèche, filtre et évapore. On obtient 0,3296g de produit recherché.

### Stade B : (3aS,4R,7S,9R,10R,11R,13R,15R,15aR,18S)-4-etyl-7-fluoro-3a,4,10,11,12,13,15,15a-octahydro-18-[[[(4-quinoléinyl)carbonyl]oxy]méthyl]-11-méthoxy-3a,7,9,11,13,15-hexaméthyl-10-[[2-O-acétyl-3,4,6-tridéoxy-3-(diméthylamino)-.béta.-D-xylo-hexopyranosyl]oxy]-14,1-(nitriloéthano)-2H-oxacyclotétradêcino[4,3-d]oxazole-2,6,8(7H,9H)-trione

On porte au reflux pendant 5 h 30 un mélange de 180 mg de produit du stade A, 6 cm³ de chlorure de méthylène, 137 µl de TEA, 0,142g de chlorure de l'acide et 33,2mg de DMAP. On verse sur une solution aqueuse d'ammoniaque à 10 %. On décante. On lave la phase organique avec une solution saturée de chlorure de sodium et à l'eau. On extrait la phase aqueuse à l'acétate d'éthyle. On rassemble les phases organiques, les séche, filtre et évapore. On obtient 0,23 g du produit recherché brut que l'on purifie par chromatographie sur silice en éluant avec le mélange chloroforme, alcool isopropylique, ammoniaque 96-4-o,1 %.

### Stade C :(3aS,4R,7S,9R,10R,11R,13R,15R,15aR,18S)-4-etyl-7-fluoro-3a,4,10,11,12,13,15,15a-octahydro-18-[[[(4-quinoleinyl)carbonyl]oxy]méthyl]-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione.

On agite pendant 24 heures un mélange de 0,135g de produit du stade précédent et 2 cm³ de méthanol. On évapore à sec. On reprend à l'acétate d'éthyle, ajoute 20 cm³ d'ammoniaque à 10 %. On agite pendant 10 minutes. On décante, extrait à l'acétate d'éthyle séche, filtre et évapore. On reprend à l'éther, filtre et sèche. On obtient ainsi le produit recherché rf = 0,40 CHCl₃, MeOH, NH₄ OH = 96-4-0,4 spectre de masse MH⁺ = 683⁺

### EXEMPLE DE COMPOSITION PHARMACEUTIQUE

On a préparé des comprimés renfermant :

| | |
|---|---|
| Produit de l'exemple 1 | 150 mg |
| Excipient q.s.p. | 1 g |

Détail de l'excipient : amidon, talc,
stéarate de magnésium

### ETUDE PHARMACOLOGIQUE DES PRODUITS DE L'INVENTION

### Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en microgrammes/cm³.

Les résultats suivants ont été obtenus : (lecture après 24 heures)

| Souches bactériennes à GRAM⁺ | Exemple 1 | Exemple 3 |
|---|---|---|
| S. aureus 011UC4 | 0,150 | 0,040 |
| S. aureus 011UC4 + sérum 50 % | 0,040 | 0,040 |
| S. aureus 011G025i | 0,600 | 0,040 |
| S. epidermidis 012G011i | 0,300 | 0,150 |
| S. pyogenes 02A1UC1 | 0,040 | ≤0,02 |
| S. agalactiae 02B1HT1 | ≤ 0,02 | 0,02 |
| S. faecalis 02D2UC1 | 0,040 | 0,02 |
| S. faecium 02D3HT1 | ≤ 0,02 | 0,02 |
| Streptococcus gr. G 02GOGR5 | 0,040 | 0,02 |
| S. mitis 02MitCBl | 0,040 | 0,02 |
| S. agalactiae 02B1SJ1c | 1,200 | 0,02 |
| S. pneumoniae 032UC1 | 0,080 | 0,02 |
| S. pneumoniae 030GR20 | ≤ 0,02 | 0,02 |

De plus, le produit des exemples 1 a montré une activité intéressante sur les souches bactériennes à gram^{⊖} suivantes : Haemophilus Influenzae 351HT3, 351CB12 et 351CA1.

## Revendications

1. Les composés de formule (I) : 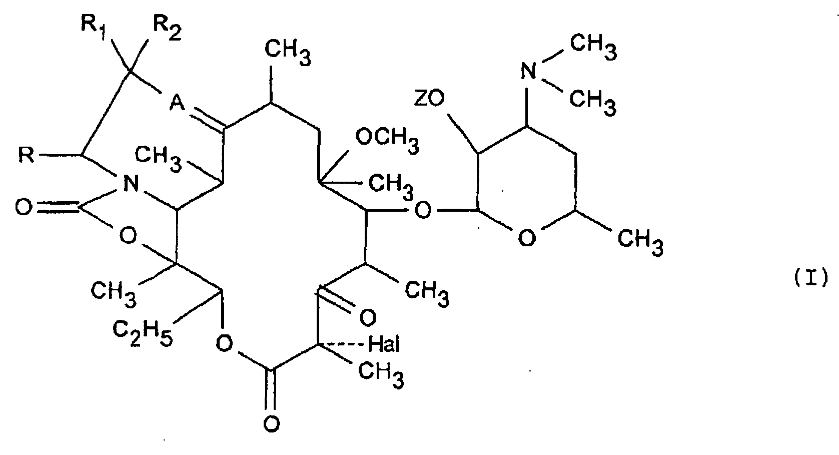 dans lesquels A représente un atome d'azote ou un groupement N->O, R₁ et R₂, identiques ou différents représentent un atome d'hydrogène ou un radical alkyle, renfermant jusqu'à 18 atomes de carbone, R représente un radical (CH₂)ₘOB dans lequel m représente un nombre entier compris entre 1 et 8 et B représente un atome d'hydrogène ou un radical COAr ou un radical (CH₂)ₙAr, n représentant un nombre entier compris entre 1 et 8 et Ar représentant un radical aryle ou hétéroaryle mono ou polycyclique et Z représente un atome d'hydrogène ou le reste d'un radical acyle renfermant jusqu'à 18 atomes de carbone, ainsi que leurs sels d'addition avec les acides.

2. Les composés de formule (I) définis à la revendication 1 dans lesquels R₁ et R₂ représentent un atome d'hydrogène.

3. Les composés de formule (I) définis à la revendication 1 ou 2, dans lesquels A représente un atome d'azote.

4. Les composés de formule (I) définis à l'une quelconque des revendications 1 à 3, dans lesquels Hal représente un atome de fluor.

5. Les composés de formule (I) définis à l'une quelconque des revendications 1 à 4, dans lesquels R représente un radical CH₂OH.

6. Les composés de formule (I) définis à la revendication 1 dont les noms suivent :
(3aS,4R,7S,9R,10R,11R,13R,15R,15aR,18S)-4-atyl-7-fluoro-3a,4,10,11,12,13,15,15a-octahydro-18-(hydroxyméthy1)-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione, et (3aS,4R,7S.9R,10R,11R,13R,15R,15aR,18S)-4-etyl-7-fluoro-3a,4,10,11,12,13,15,15a-octahydro-18-[[[(4-quinoleinyl)carbonyl]oxy]méthyl]-11-méthoxy-3a,7,9,11,13,15-hexaméthyl-10-[[3,4,6-tridéoxy-3-(diméthylamino)-.béta.-D xylo-hexopyranosyl]oxy]-14,1-(nitriloéthano)-2H-oxacyclotétradécino[4,3-d]oxazole-2.6,8(7H,9H)-trione.

7. Composé de formule (I) défini à la revendication 1 **caractérisé en ce qu'**il s'agit de la (3aS,4R,7S,9R,10R,11R,13R,15R,15aR,18S)-4-etyl-7-fluoro-3a,4,10,11,12,13,15,15a-octahydro-18-(hydroxyméthyl)-11-méthoxy-3a,7,9,11,13,15-hexaméthyl-10-[[3,4,6-tridéoxy-3-(diméthylamino)-.béta.-D-xylo-hexopyranosyl]oxy]-14,1-(nitriloéthano)-2H-oxacyclotétradécino[4,3-d]oxazole-2,6,8(7H,9H)-trione.

8. A titre de médicaments, les composés selon l'une quelconque des revendications 1 à 5, ainsi que leurs sels pharmaceutiquement acceptables.

9. A titre de médicaments, les composés de formule (I) définis à la revendication 6 ou 7, ainsi que leurs sels pharmaceutiquement acceptables.

10. Les compositions pharmaceutiques renfermant comme principe actif au moins un médicament défini à la revendication 8 ou 9.

11. Procédé de préparation des composés selon l'une quelconque des revendications 1 à 7, **caractérisé en ce que** l'on soumet un composé de formule (II) : 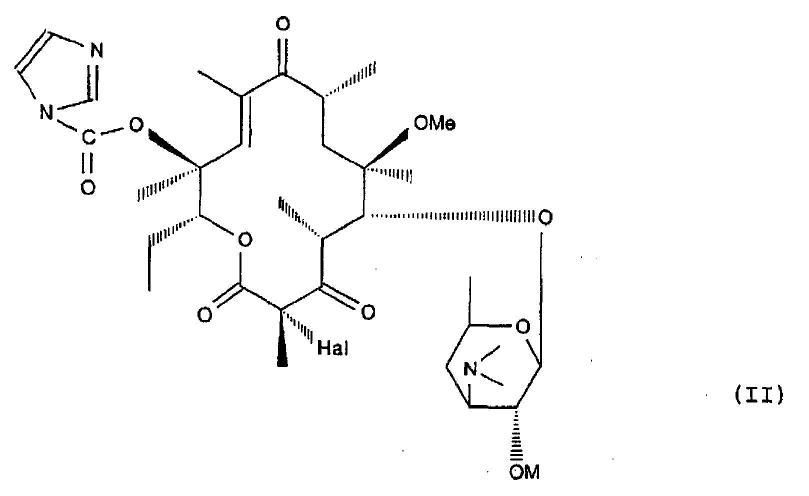 dans laquelle Hal représente un atome d'halogène, OM représente un groupement hydroxyle bloqué, à l'action d'un composé de formule (III) : 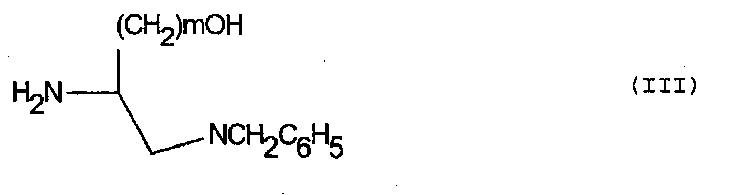 dans laquelle m représente un nombre entier compris entre 1 et 8, pour obtenir le composé de formule (IV) : 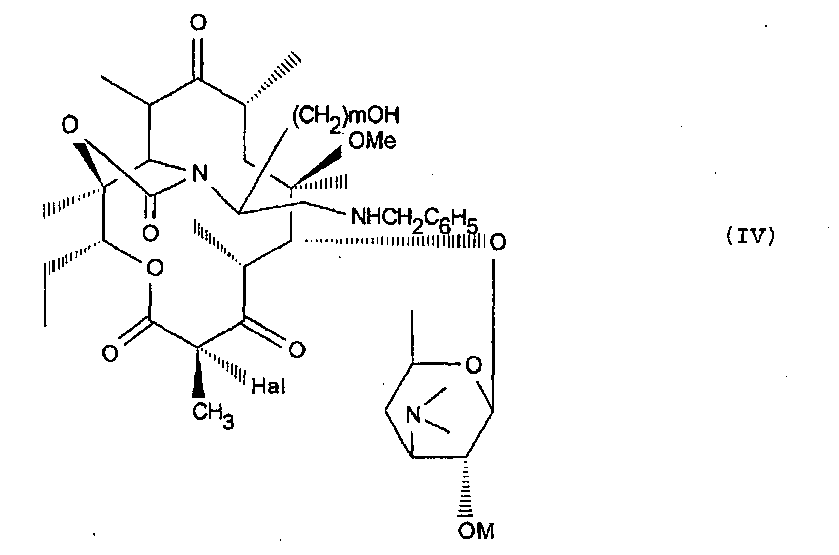 puis libère l'hydroxyle en 2' pour obtenir le composé de formule (V) : 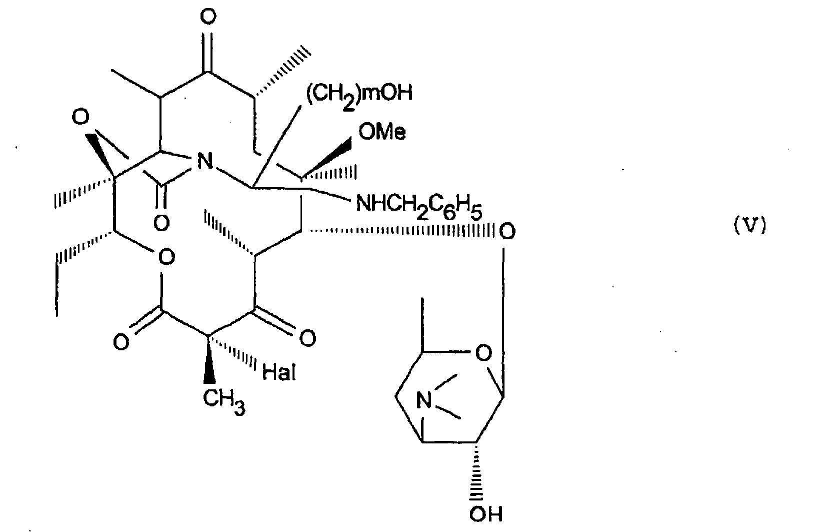 que l'on soumet à l'action d'un agent de débenzylation, pour obtenir le composé de formule (VI) : 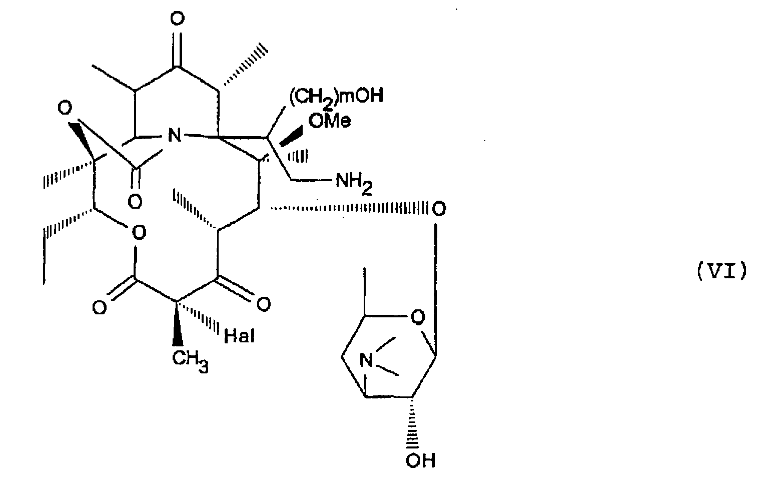 que l'on soumet à l'action d'un agent de cyclisation pour obtenir le composé de formule (IA) : 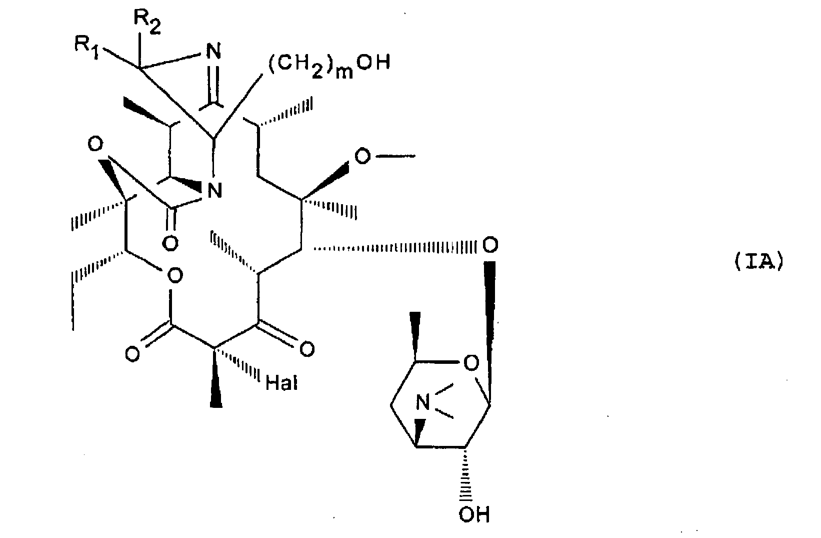 dans lequel R représente un radical (CH₂)ₘOH, que l'on soumet à l'action d'un agent d'alkylation ou d'acylation du groupement (CH₂)ₘOH pour obtenir le composé de formule (IB) correspondant das lequel B représente un groupement COAr ou (CH₂)ₙAr, puis, si désiré estérifie le groupement OH en 2' et/ou soumet à l'action d'un acide pour obtenir un sel du composé de formule (I) obtenu.

12. A titre de produits chimiques nouveaux, les composés de formules (IV), (V) et (VI) définis à la revendication 11.

13. Variante du procédé selon la revendication 11, **caractérisé en ce que** l'on soumet un composé de formule (IIIA) : 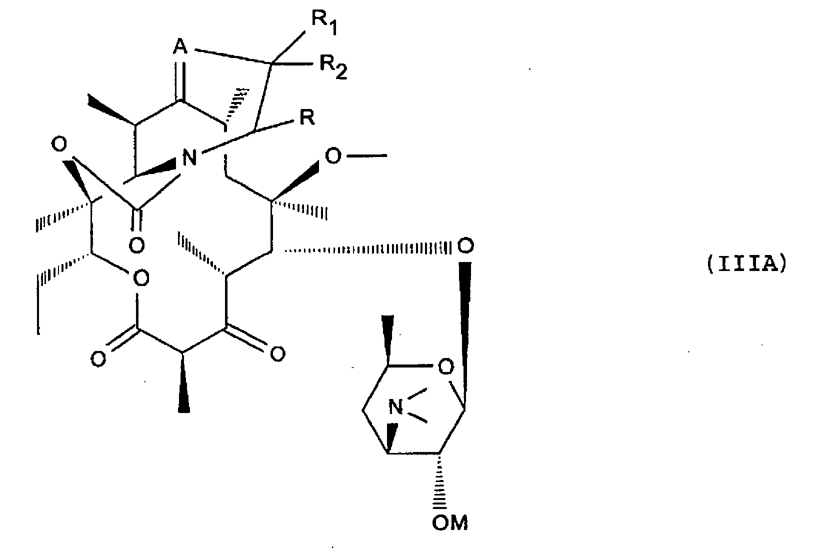 dans laquelle A, R, R₁ et R₂ conservent leur signification précédente et OM représente un groupement hydroxyle bloqué, à l'action d'un agent d'halogénation pour obtenir le composé de formule (IB) : 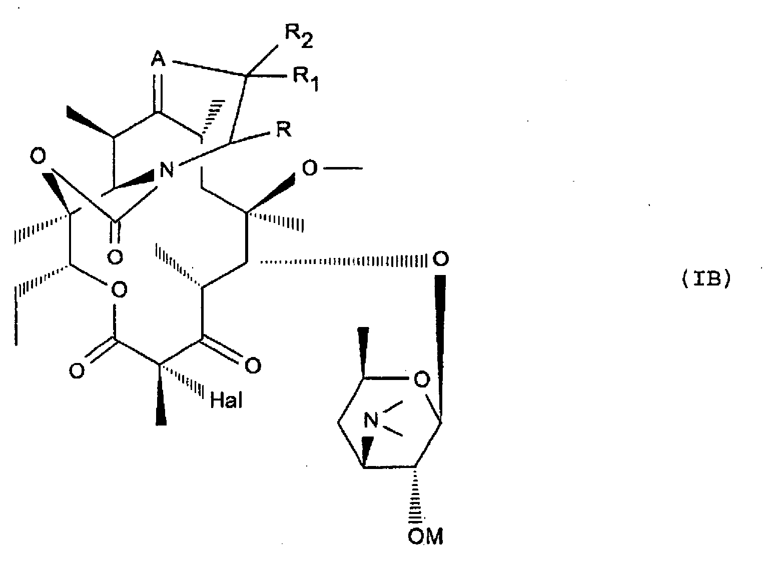 que l'on soumet si désiré, à l'action d'un agent de libération du groupement hydroxyle en 2', pour obtenir le composé de formule (I) correspondant dans lequel Z est un atome d'hydrogène que l'on soumet, si désiré à l'action d'un agent d'estérification du groupe OH en 2' ou à l'action d'un acide pour en former le sel.

## Claims

1. Compounds of formula (I) ; 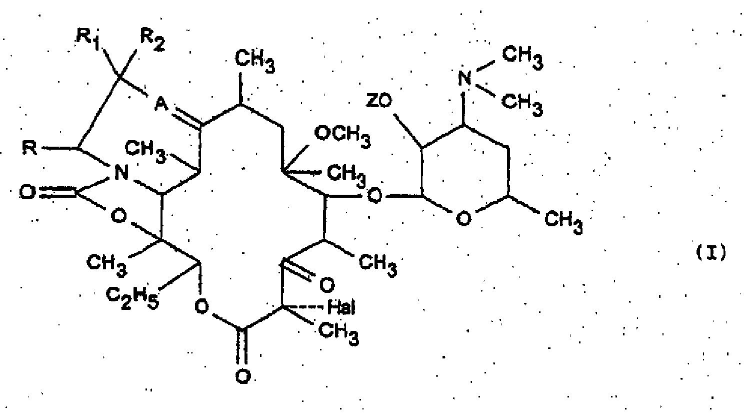 in which A represents a nitrogen atom or an N→O group, R₁ and R₂, which are identical or different, represent a hydrogen atom or an alkyl radical including up to 18 carbon atoms, R represents a (CH₂)ₘOB radical in which m represents an integer between 1 and 8 and B represents a hydrogen atom or a COAr radical or a (CH₂)nAr radical, n representing an integer between 1 and 8 and Ar representing a mono- or polycyclic aryl or heteroaryl radical, and Z represents a hydrogen atom or the residue of an acyl radical including up to 18 carbon atoms, and their addition salts with acids.

2. The compounds of formula (I) defined in Claim 1, in which R₁ and R₂ represent a hydrogen atom.

3. The compounds of formula (I) defined in Claim 1 or 2, in which A represents a nitrogen atom.

4. The compounds of formula (I) defined in any one of Claims 1 to 3, in which Hal represents a fluorine atom.

5. The compounds of formula (I) defined in any one of Claims 1 to 4, in which R represents a CH₂OH radical.

6. The compounds of formula (I) defined in Claim 1, the names of which follow:
(3aS,4R,75,9R,10R,11R,13R,15R,15aR,18S)-4-ethyl-7-fluoro-3a,4,10,11,12,13,15,15a-octahydro-18-(hydroxymethyl)-11-methoxy-3a,7,9,11,13,15-hexamethyl-10-[[3,4,6-trideoxy-3-(dimethylamino)-β-D-xylo-hexopyranosyl]oxy]-14,1-(nitriloethano)-2H-oxacyclotetradecino[4,3-d]oxazole-2,6,8(7H,9H)-trione and
(3aS,4R,7S,9R,10R,11R,13R,15R,15aR,18S)-4-ethyl-7-fluoro-3a,4,10,11,12,13,15,15a-octahyaro-Z8-[[[(4-quinolyl)carbonyl]oxy]methyl]-11-methoxy-3a,7,9,11,13,15-hexamethyl-10-[[3,4,6-trideoxy-3-(dimethylamino)-3-D-xylo-hexopyranosyl]oxy]-14,1-(nitriloethano)-2H-oxacyclotetradecino[4,3-d]oxazole-2,5,8(7H,9H)-trione.

7. Compound of formula (I) defined in Claim 1, **characterized in that** it is (3aS,4R,7S,9R,10R,11R,13R,15R,15aR,18S)-4-ethyl-7-fluoro-3a,4,10,11,12,13,15,15a-octahydro-18-(hydroxymethyl)-11-methoxy-3a,7,9,11,13,15-hexamethyl-10-[[3,4,6-trideoxy-3-(dimethylamino)-β-D-xylo-hexopyranosyl]oxy]-14,1-(nitriloethano)-2H-oxacyclotetradecino[4,3-d]oxazole-2,6,8(7H,9H)-trione.

8. As medicaments, the compounds according to any one of Claims 1 to 5 and their pharmaceutically acceptable salts.

9. As medicaments, the compounds of formula (I) defined in Claim 6 or 7 and their pharmaceutically acceptable salts.

10. The pharmaceutical compositions including, as active principle, at least one medicament defined in Claim 8 or 9.

11. Process for the preparation of the compounds according to any one of Claims 1 to 7, **characterized in that** a compound of formula (II): 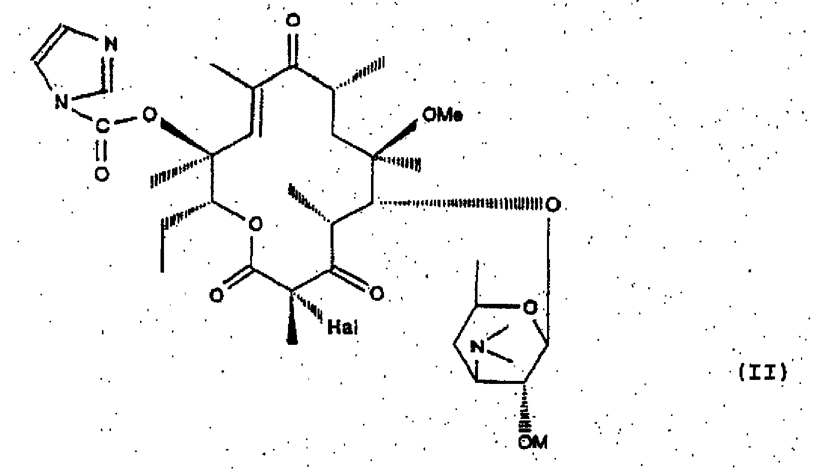 in which Hal represents a halogen atom and OM represents a protected hydroxyl group, is subjected to the action of a compound of formula (III): 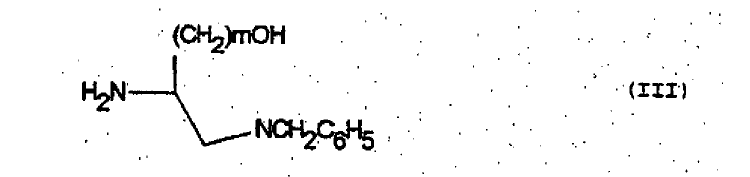 in which m represents an integer between 1 and 8, in order to obtain the compound of formula (IV): 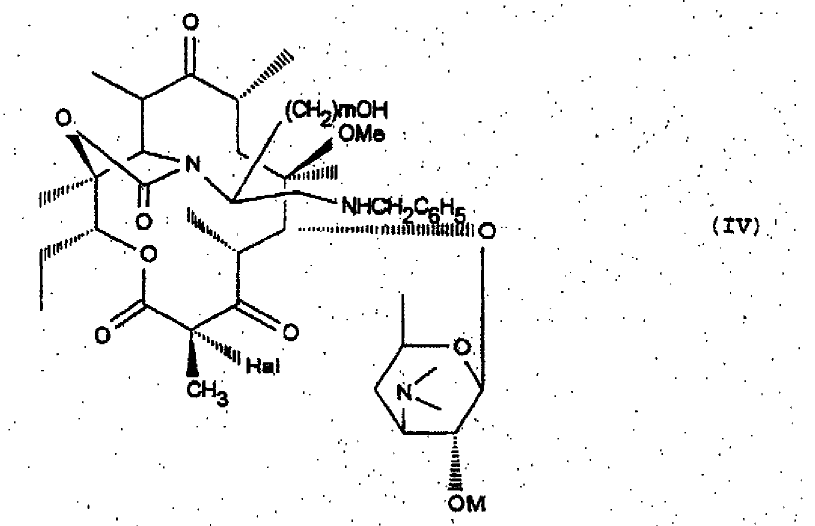 then the hydroxyl in the 2' position is released, in order to obtain the compound of formula (V): 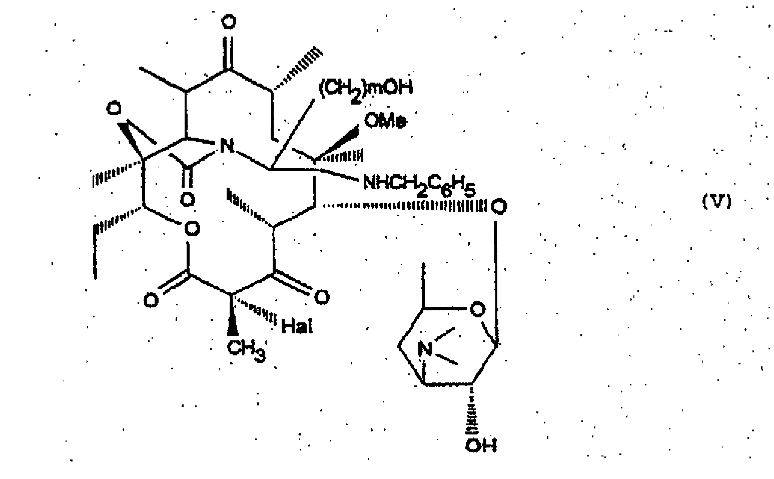 which is subjected to the action of a debenzylating agent, in order to obtain the compound of formula (VI): 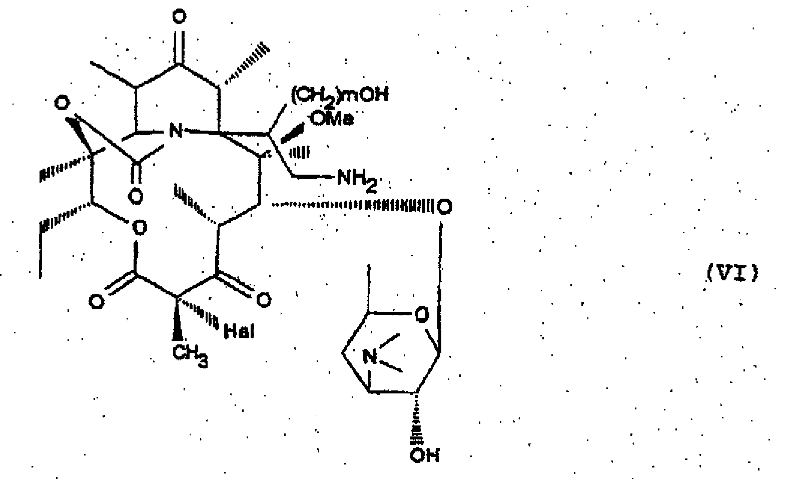 which is subjected to the action of a cyclizing agent, in order to obtain the compound of formula (IA): 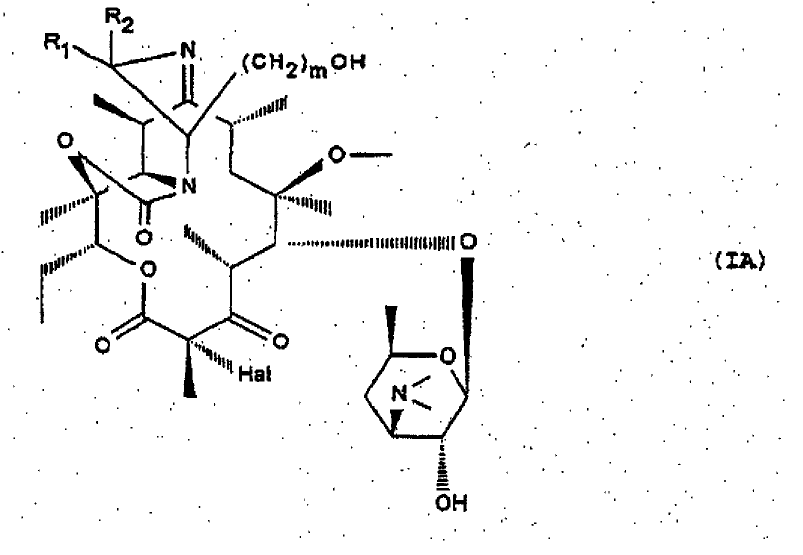 in which R represents a (CH₂)ₘOH radical, which is subjected to the action of an agent for alkylating or acylating the (CH₂)ₘOH group, in order to obtain the corresponding compound of formula (IB) in which B represents a COAr or (CH₂)ₙAr group, and then, if desired, the OH group in the 2' position is esterified and/or the product is subjected to the action of an acid, in order to obtain a salt of the compound of formula (I) obtained.

12. As novel chemical products, the compounds of formulae (IV), (V) and (VI) defined in Claim 11.

13. Alternative form of the process according to Claim 11, **characterized in that** a compound of formula (IIIA) : 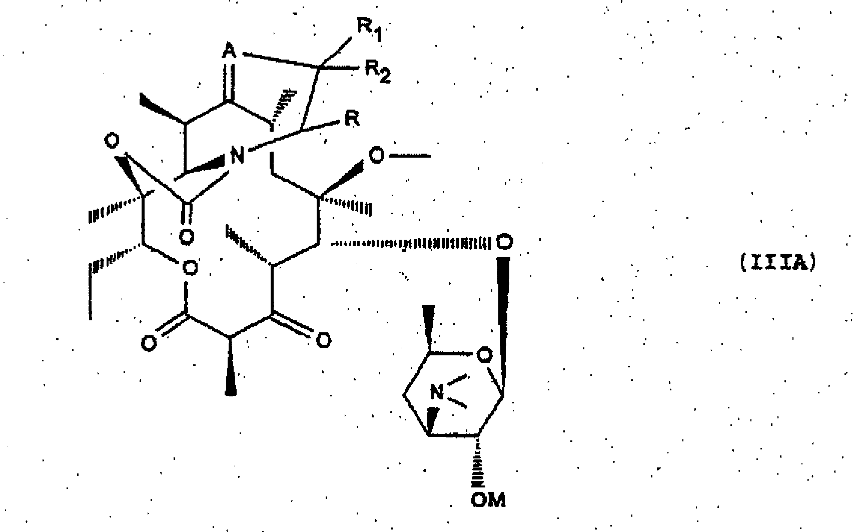 in which A, R, R₁ and R₂ retain their above meanings and OM represents a protected hydroxyl group, is subjected to the action of a halogenating agent, in order to obtain the compound of formula (IB) : 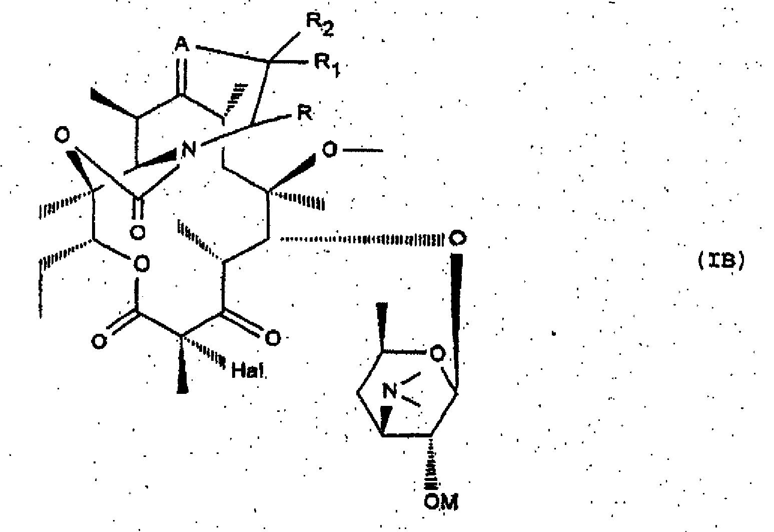 which is subjected, if desired, to the action of an agent for the release of the hydroxyl group in the 2' position, in order to obtain the corresponding compound of formula (I) in which Z is a hydrogen atom, which is subjected, if desired, to the action of an agent for the esterification of the OH group in the 2' position or to the action of an acid, in order to form the salt thereof.

## Patentansprüche

1. Verbindungen der Formel (I): 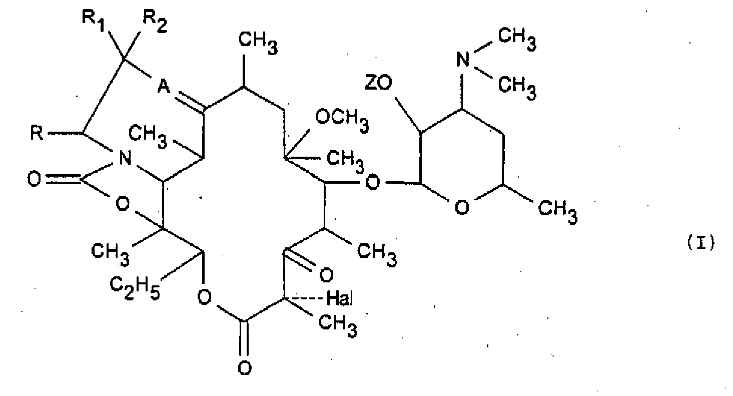 worin A für ein Stickstoffatom oder eine N->O-Gruppe steht, R₁ und R₂ gleich oder verschieden sind und für ein Wasserstoffatom oder einen Alkylrest mit bis zu 18 Kohlenstoffatomen steht, R für einen (CH2)ₘOB-Rest steht, worin m für eine ganze Zahl zwischen 1 und 8 steht und B für ein Wasserstoffatom oder einen COAr-Rest oder einen (CH₂)ₙ-Ar-Rest steht, wobei n für eine ganze Zahl zwischen 1 und 8 steht und Ar für einen mono- oder polycyclischen Aryl- oder Heteroarylrest steht, und Z für ein Wasserstoffatom oder den Rest eines Acylrests mit bis zu 18 Kohlenstoffatomen steht, sowie Säureadditionssalze davon.

2. Verbindungen der Formel (I) nach Anspruch 1, worin R₁ und R₂ für ein Wasserstoffatom stehen.

3. Verbindungen der Formel (I) nach Anspruch 1 oder 2, worin A für ein Stickstoffatom steht.

4. Verbindungen der Formel (I) nach einem der Ansprüche 1 bis 3, worin Hal für ein Fluoratom steht.

5. Verbindungen der Formel (I) nach einem der Ansprüche 1 bis 4, worin R für einen CH₂OH-Rest steht.

6. Verbindungen der Formel (I) nach Anspruch 1 mit den folgenden Namen:
(3aS,4R,7S,9R,10R,11R,13R,15R,15aR,18S)-4-Ethyl-7-fluor-3a,4,10,11,12,13,15,15a-actahydro-18-(hydroxymethyl)-11-methoxy-3a,7,9,11,13,15-hexamethyl-10-[[3,4,6-tridesoxy-3-(dimethylamino)-beta-D-xylohexopyranosyl]oxy]-14,1-(nitriloethano)-2H-oxacyclotetradecino[4,3-d]oxazol-2,6,8(7H,9H)-trion und (3aS,4R,7S,9R,10R,11R,13R,15R,15aR,18S)-4-Ethyl-7-fluor-3a,4,10,11,12,13,15,15a-octahydro-18-[[[(4-chinolinyl)carbonyl]oxy]methyl]-11-methoxy-3a,7,9,11,13,15-hexamethyl-10-[[3,4,6-tridesoxy-3-(dimethylamino)-beta-D-xylohexopyranosyl]oxy]-14,1-(nitriloethano)-2H-oxacyclotetradecino[4,3-d]oxazol-2,6,8(7H,9H)-trion.

7. Verbindung der Formel (I) nach Anspruch 1, **dadurch gekennzeichnet, daß** es sich dabei um (3aS,4R,7S,9R,10R,11R,13R,15R,15aR,18S)-4-Ethyl-7-fluor-3a,4,10,11,12,13,15,15a-octahydro-18-(hydroxymethyl)-11-methoxy-3a,7,9,11,13,15-hexamethyl-10-[[3,4,6-tridesoxy-3-(dimethylamino)-beta-D-xylohexopyranosyl]oxy]-14,1-(nitriloethano)-2H-oxacyclotetradecino[4,3-d]oxazol-2,6,8(7H,9H)-trion handelt.

8. Verbindungen nach einem der Ansprüche 1 bis 5 sowie ihre pharmazeutisch unbedenklichen Salze als Medikamente.

9. Verbindungen der Formel (I) nach Anspruch 6 oder 7 sowie ihre pharmazeutisch unbedenklichen Salze als Medikamente.

10. Pharmazeutische Zusammensetzungen, die als Wirkstoff mindestens ein Medikament nach Anspruch 8 oder 9 enthalten.

11. Verfahren zur Herstellung der Verbindungen nach einem der Ansprüche 1 bis 7, **dadurch gekennzeichnet, daß** man eine Verbindung der Formel (II): 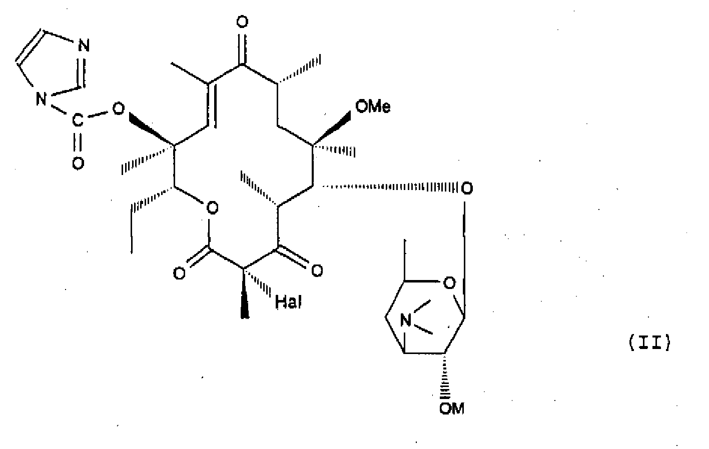 worin Hal für ein Halogenatom steht und OM für eine blockierte Hydroxylgruppe steht, mit einer Verbindung der Formel (III): 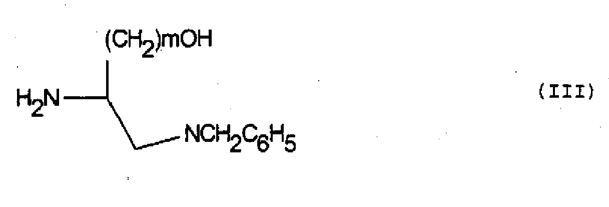 worin m für eine ganze Zahl zwischen 1 und 8 steht, zu der Verbindung der Formel (IV): 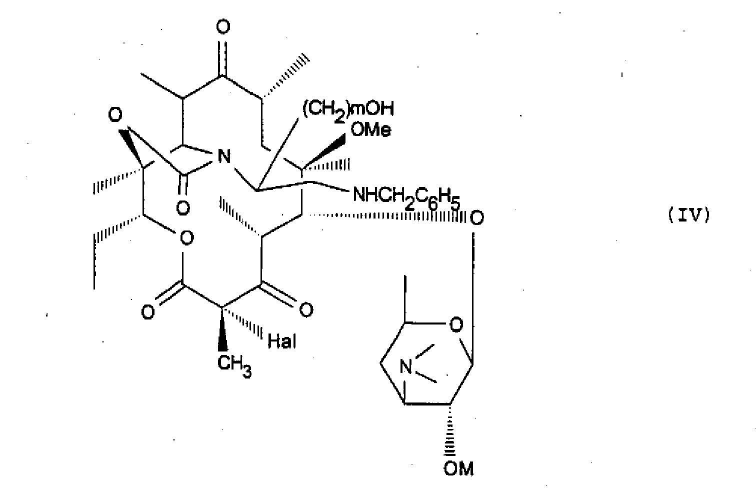 umsetzt und dann das Hydroxyl in 2'-Stellung entschützt, wobei man die Verbindung der Formel (V): 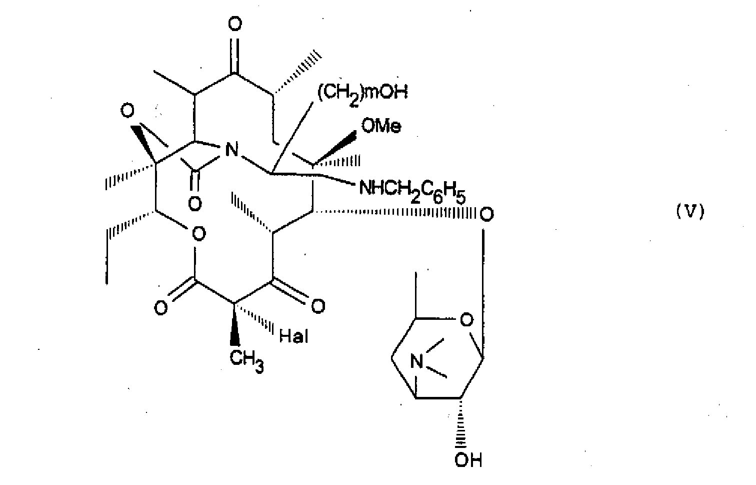 erhält, die man mit einem Debenzylierungsmittel zu der Verbindung der Formel (VI): 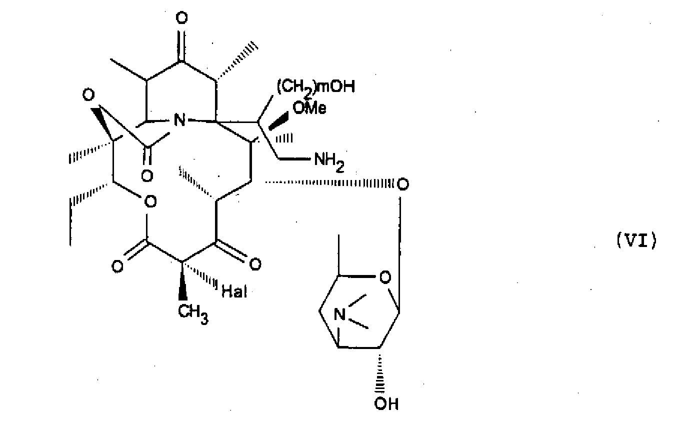 umsetzt, die man mit einem Cyclisierungsmittel zu der Verbindung der Formel (IA): 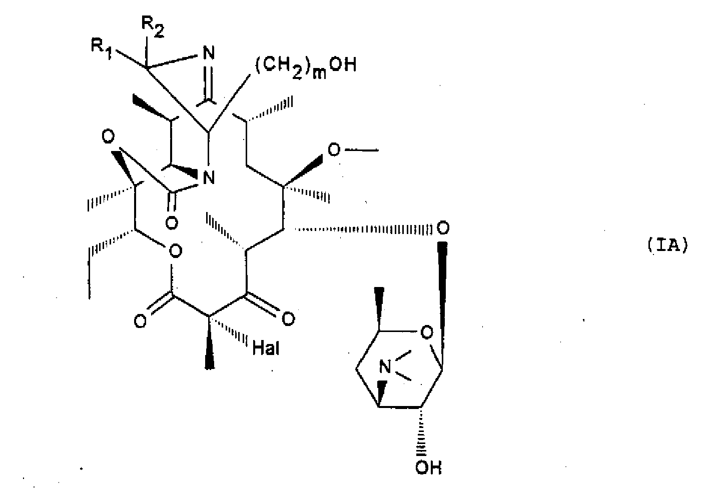 worin R für einen (CH₂)ₘOH-Rest steht, umsetzt, die man mit einem Mittel zur Alkylierung oder Acylierung der (CH₂)ₘOH-Gruppe zu der entsprechenden Verbindung der Formel (IB), worin B für eine COAr- oder (CH₂)ₙAr-Gruppe steht, umsetzt und dann gegebenenfalls die OH-Gruppe in 2`-Stellung verestert und/oder das Produkt mit einer Säure zu einem Salz der erhaltenen Verbindung der Formel (I) umsetzt.

12. Verbindungen der Formeln (IV), (V) und (VI) nach Anspruch 11 als neue chemische Produkte.

13. Variante des Verfahrens nach Anspruch 11, **dadurch gekennzeichnet, daß** man eine Verbindung der Formel (IIIA): 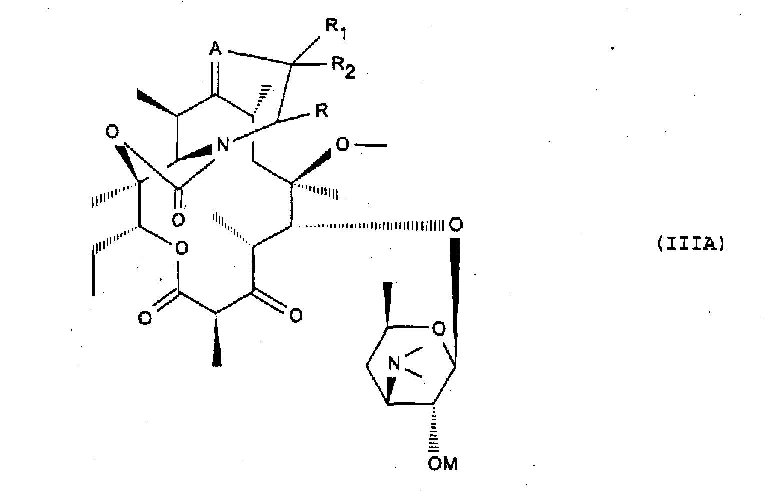 worin A, R, R₁ und R₂ ihre vorhergehende Bedeutung behalten und OM für eine blockierte Hydroxylgruppe steht, mit einem Halogenierungsmittel zu der Verbindung der Formel (IB): 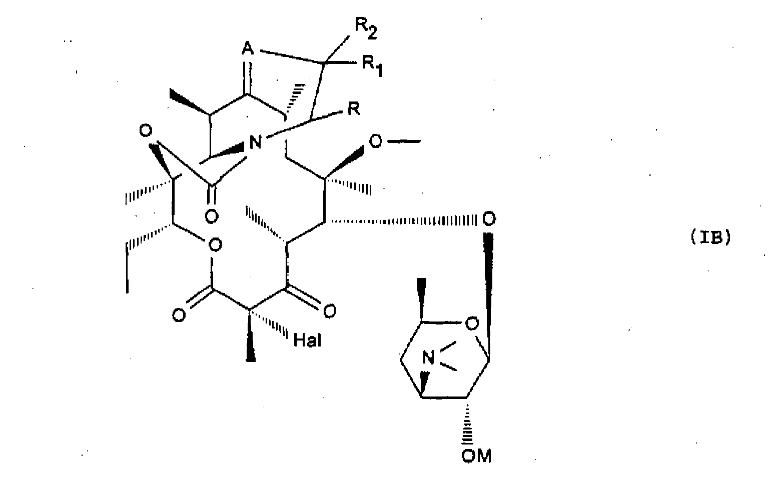 umsetzt, die man gegebenenfalls mit einem Mittel zur Entschützung der Hydroxylgruppe in 2'-Stellung zu der entsprechenden Verbindung der Formel (I), worin Z für ein Wasserstoffatom steht, umsetzt, die man gegebenenfalls mit einem Mittel zur Veresterung der OH-Gruppe in 2'-Stellung oder mit einer Säure zur Bildung des Salzes umsetzt.
